# EUROPEAN PATENT APPLICATION

(11) **EP 4 700 701 A1**
(43) Date of publication of application: **25.02.2026**
(21) Application number: 24195407.2
(22) Date of filing: 20.08.2024
(51) Int. Cl.: G06T 7/10, G06V 10/82, G16H 30/40

(54) **REPLACEMENT OF A REGION OF INTEREST CONTAINING AN ABNORMAL ANATOMICAL STRUCTURE**

(71) Applicant: Koninklijke Philips N.V., 5656 AG Eindhoven (NL)
(72) Inventor: SCHNELLBÄCHER, Nikolas David, Eindhoven (NL); GRASS, Michael, Eindhoven (NL)
(74) Representative: Philips Intellectual Property & Standards

(57) **Abstract**

Disclosed herein is a computer implemented method of medical imaging, the method comprising: receiving (200) an acquired medical image (130) descriptive of an internal anatomy of a subject (402); receiving (202) a selection of a region of interest (132) of the acquired medical image containing an abnormal anatomical structure (404); generating (204) an ensemble (140) of generated medical images (134, 136, 138) using one or more generative inpainting models (122, 124, 126) to replace the region of interest; and displaying (206) two or more images selected from the acquired medical image and the ensemble of generated medical images.

## Description

### FIELD OF THE INVENTION

The invention relates to medical images of internal anatomy, in particular to the segmentation of medical images.

### BACKGROUND OF THE INVENTION

The detection of abnormal anatomical structures such as lesions may be hard to detect in medical images because there may be weak contrast or image intensity in comparison to neighboring tissue. This for example may make the radiological reading of many pathologies both challenging and error prone.

International patent application WO2023021076A1 discloses generating ground truth data for training a machine-learning model, a computer-implemented method is proposed for training a machine-learning model for classifying image features in an image acquired by a medical scanner. The method comprises: receiving an image acquired by a medical scanner, identifying an artefact in the image, segmenting the artefact to obtain a segmentation mask of a region, on which to perform inpainting, performing inpainting on the region to obtained an inpainted image, and incorporating the inpainted image into a training set for the development of the machine-learning model.

### SUMMARY OF THE INVENTION

The invention provides for a computer-implemented method, a computer program product, and a medical system in the independent claims. Embodiments are given in the dependent claims.

In one aspect, the invention provides for a computer-implemented method of medical imaging. The method comprises receiving an acquired medical image descriptive of an internal anatomy of a subject. The method further comprises receiving a selection of a region of interest of the image containing an abnormal anatomical structure. The method further comprises generating an ensemble of generated medical images using one or more generative inpainting models to replace the region of interest. The method further comprises displaying two or more images selected from the medical image and the ensemble of generated medical images.

In another aspect, the invention provides for a computer program product comprising a computer-readable storage medium having computer-readable program code embodied therewith. The computer-readable program code is configured to implement the computer-implemented method.

In another aspect, a medical system is disclosed. The medical system comprises a memory storing machine-executable instructions and one or more generative inpainting models. The medical system further comprises a computational system. Execution of the machine-executable instructions causes the computational system to receive an acquired medical image that is descriptive of an internal anatomy of a subject. Execution of the machine-executable instructions further causes the computational system to receive a selection of a region of interest of the image containing an abnormal anatomical structure. Execution of the machine-executable instructions further causes the computational system to generate an ensemble of generated medical images using one or more generative inpainting models to replace the region of interest. Execution of the machine-executable instructions further causes the computational system to display two or more images selected from the medical image and the ensemble of generated medical images.

### BRIEF DESCRIPTION OF THE DRAWINGS

In the following preferred embodiments of the invention will be described, by way of example only, and with reference to the drawings in which:
Fig. 1 illustrates an example of a medical system.
Fig. 2 shows a flow chart which illustrates a method of using the medical system of Fig. 1.
Fig. 3 illustrates a further example of a cloud-based medical system.
Fig. 4 illustrates an example of a user interface.

### DETAILED DESCRIPTION OF EMBODIMENTS

Like numbered elements in these figures are either equivalent elements or perform the same function. Elements which have been discussed previously will not necessarily be discussed in later figures if the function is equivalent.

In an example, there is a computer-implemented method of medical imaging. The method comprises receiving an acquired medical image descriptive of an internal anatomy of a subject. The acquired medical image may for example be of different imaging modalities such as magnetic resonance imaging, computed tomography, ultrasound, positron emission tomography, and single-photo emission tomography. The method further comprises receiving a selection of a region of interest of the image containing an abnormal anatomical structure. For example, the region of interest may define a region of the image which contains the abnormal anatomical structure. The method further comprises generating an ensemble of generated medical images using one or more generative inpainting models to replace the region of interest. In one example, there is a single generative inpainting model that is used to generate more than one generated medical images to provide the ensemble of generated medical images. In other examples, there may be multiple generative inpainting models and they are used to produce the ensemble of generated medical images. The method further comprises displaying two or more images selected from the medical image and the ensemble of generated medical images. This example may be beneficial because it may provide a means for providing at a glance an ability to understand the anatomical structure by looking at the two or more selected images.

This example may be beneficial because it may be used in further examples to provide images for a User Interface (UI) which allows a medical professional in-the-loop to mask any suspicious lesion region by specifying a ROI(s) which contains the lesions of interest. Subsequently, the viewing interface temporarily removes said ROI and creates an ensemble of generated medical images which may, for example, be plausible realizations for the image content of said ROI(s) by means of generative inpainting models. This ensemble of image realizations can, for example, be browsed with an appropriate UI either one-by-one or by computing an aggregated average realization of the said ROI(s). The generative image creation can further be configured in two modes, sampling from healthy (benign) tissue, demonstrating how (on average) the ROI would look like as healthy tissue, or sampling from malignant / pathological tissue examples, illustrating how other lesions in this anatomical region could look like. The pathologies can be implicitly inferred from the tissue context or explicitly conditioned by a corresponding user input, specifying the disease specification by a free text input. Using the healthy ensemble sampling approach, we further suggest a generative background subtraction (GBS) method, where we subtract either a single or averaged realization of healthy background from the true ROI image content, resulting in an, for example, enhanced lesions visualization.

In various examples, the inpainting may be a multi-channel inpainting used to in-paint multi-contrast images as they are generated by spectral imaging (photo, scatter, conventional), dark field (phase, dark field, conventional) or multi-contrast MR (i.e., T1, T2, Dixon Fat image, Dixon water image, proton density, or via fingerprinting).

In another example, the one or more generative inpainting models comprises a normal anatomy generative model configured for generating normal anatomical structures in the region of interest. The ensemble of generated medical images comprises an artificial abnormal structure-free image generated from the normal anatomy generative model. The artificial abnormal structure-free image is preferably generated using a combination of multiple images output by the normal anatomy generative model. In this example, an image is generated in which the abnormal anatomical structure has been removed. This may for example be useful for various things such as generating a segmentation of the abnormal anatomical structure as well as providing the means to highlight or emphasize the location of the abnormal anatomical structure when looking at it briefly.

In another example, the method further comprises generating a difference image by subtracting the acquired medical image and the artificial abnormal structure-free image from each other and then generating an image segmentation of the abnormal anatomical structure using the difference image. This example may be beneficial because it may provide a means of segmenting an abnormal anatomical structure without specifically training it to segment this particular type of abnormal structure. When the difference image is produced it may be emphasized in the difference image where this abnormal anatomical structure is. The image segmentation may for example be easily limited by thresholding the difference image.

The normal anatomy generative model may for example be trained by using images of normal anatomical structures from acquired medical images which are used as training data. For example, various types of GAN or diffusion type models may be used for implementing this.

In another example, the method further comprises displaying a superposition of the image segmentation on the acquired medical image. This may be beneficial because it may provide for an improved means of segmenting the acquired medical image. As was mentioned above, because the normal anatomy generative model is able to replace the abnormal anatomical structure in general, this provides a means of segmenting the acquired medical image without specialized training for every type of tumor or abnormal structures.

In another example, the abnormal anatomical structure is one or more tumors. This example may be beneficial because the tumors may come in a variety of shapes and sizes which would be difficult to train a specialized segmentation algorithm or model for. This example is beneficial because the method enables a generalized means of segmenting one or more tumors automatically.

In another example, the method further comprises generating a predictive image in response to inputting the acquired medical image into a tumor progression model configured for predicting tumor growth after a predetermined time period. The predicted image is used for determining the image segmentation instead of the acquired medical image. This example may be beneficial because it may provide for a means of identifying a tissue which may become cancerous or contain tumorous tissue. This may for example be useful in advanced screening for the progression of tumors as well as during various therapy operations, such as radiation therapy, proton therapy, surgery (for example with surgical robots), ablation (microwave, radiofrequency), and other modalities. For example, if it is known or predicted into what regions a tumor would likely expand, one can then irradiate or otherwise treat these regions with the anticipation that they have a high likelihood of containing tumor tissue in the future.

The tumor progression model that takes a medical image, such as an MRI or CT image, as input and outputs an image that predicts the growth of a tumor after a predetermined time period, one could utilize advanced neural network architectures such as Convolutional Neural Networks (CNNs) and Recurrent Neural Networks (RNNs). Specifically, a 3D CNN could be employed to effectively capture the spatial features of the medical images, as these networks are adept at processing volumetric data. The 3D CNN would extract high-level features from the medical images, which can then be fed into a Long Short-Term Memory (LSTM) network or a Gated Recurrent Unit (GRU) network to model the temporal progression of the tumor. This combination leverages the spatial recognition power of CNNs with the temporal processing capabilities of RNNs.

The training process may involve several steps. First, a comprehensive dataset of annotated medical images, including corresponding tumor growth measurements over time, may be collected. The dataset may be split into training, validation, and test sets to ensure robust model evaluation. Data augmentation techniques, such as rotation, scaling, and flipping, can be applied to enhance the diversity of the training data and improve model generalization. The 3D CNN can be pre-trained on a large dataset of medical images to capture general features and then fine-tuned on the specific tumor progression dataset. During training, the LSTM or GRU network is trained alongside the 3D CNN to learn the temporal patterns of tumor growth. The model's performance can be optimized using loss functions such as Mean Squared Error (MSE) or Mean Absolute Error (MAE), and techniques like early stopping, learning rate scheduling, and regularization can be employed to prevent overfitting. The trained model can then be evaluated on the test set to assess its predictive accuracy and generalization capability. Depending upon the training (the annotated medical images used), the tumor progression model may in some examples also or alternatively predict changes in organ or other tissue shape in response the growth of a tumor.

In another example, the method further comprises generating therapy system control commands using the acquired medical image and the image segmentation. The therapy control commands are configured for controlling a therapy system to modify the abnormal anatomical structure. The therapy system could for example by a radiotherapy system, a HIFU system, an RF ablation device, a surgical robot, a Da-Vinci surgical robot, or other therapy or surgical device. This example may be beneficial because the image segmentation may be more accurate than can be generated using conventional algorithms. When the predicted image is used for making the image segmentation it may be particularly beneficial because it may be used to ablate or modify tissue that is likely to become tumorous or cancerous in the future. This may for example lead to better therapy using the therapy system.

In another example, the method comprises controlling a therapy system with the therapy control commands.

With the proviso that methods described herein exclude the treatment or surgery of a human or animal body. For example, controlling the therapy system could be limited to sending the therapy control commands to the therapy system and not encompass the actual act or method of performing the modification or surgery on the human or animal body. Any reference to surgical methods within this application is provided for illustrative purposes only and does not constitute a claim for patent protection of such methods.

In another example, the one of more generative inpainting models further comprises an alternative inpainting model configured for replacing the region of interest with the same type of abnormal anatomical structure. This may for example be beneficial because it may provide an alternative view which may assist in the recognition of the type of abnormal structure.

In another example, the one or more generative inpainting models further comprises a replacement inpainting model configured to replace the region of interest with at least one different type of abnormal anatomical structure. For example, it may replace it with a different type of abnormal anatomical structure, and this may be useful in recognizing if the anatomical structure is more similar or different to one of these alternative structures.

In another example, the one or more generative inpainting models comprises any one of the following: diffusion inpainting models, GAN inpainting models, and combinations thereof. These may for example be useful in providing a means of replacing the region of interest either with normal anatomical structures or different types of abnormal anatomical structures.

The diffusion inpainting models may be implemented in a variety of different ways. A neural network such as a U-Net or ResNet may be trained to a particular target type of anatomical abnormal structure or a lack of abnormal anatomical structures. These models are then subjected to noise and the diffusion inpainting model is trained to remove this noise which is added to the image. Once trained, the diffusion inpainting model may then repeatedly use this U-Net or ResNet to replace the removed area with an image that fits the rest of the model.

In general, to train a diffusion model to replace a portion of a medical image such as a CT or MRI image with normal tissue, a different version of the same pathology, or a different pathology, one could use types of diffusion models such as Denoising Diffusion Probabilistic Models (DDPMs) and Denoising Score Matching with Langevin Dynamics (SMLD).

DDPMs are a class of generative models that learn to model the data distribution by reversing a diffusion process that gradually adds noise to the data until it becomes pure noise. SMLD, on the other hand, relies on score matching to estimate the gradients of the data distribution, followed by Langevin dynamics to sample from this distribution. Both types are suitable for generating high-quality, realistic images and can be adapted for medical image translation tasks.

To train these diffusion models, first, gather a large dataset of medical images, ensuring a diverse representation of normal tissues and various pathologies. Preprocess these images to ensure consistency in size and format and normalize the pixel values to a suitable range. Define the neural network architecture used for the denoising function in the diffusion model. Typically, a U-Net architecture is employed due to its effectiveness in capturing multi-scale features, which is crucial for medical images.

Next, implement the forward diffusion process, where Gaussian noise is gradually added to the images over several time steps. This process is parameterized to ensure the noise addition is controllable and reversible. The reverse diffusion process is then modeled using the neural network, which is trained to denoise the images at each time step. This involves minimizing a loss function that measures the difference between the denoised images and the original images before noise was added. For DDPMs, this typically involves optimizing a variational lower bound on the data likelihood, while for SMLD, it involves optimizing the score-matching objective.

The training process involves iterating over the dataset, gradually learning to reverse the noise addition process. This requires substantial computational resources and time, as the model needs to learn to denoise images progressively. Techniques such as data augmentation, early stopping, and learning rate scheduling can be used to improve training efficiency and model performance. Once the model is trained, it can be evaluated using a separate validation set to ensure the generated images are of high quality and medically relevant.

In implementing a GAN inpainting model the GAN is trained to replace a region of an image with either normal anatomical structures or various types of abnormal anatomical structures. To train a Generative Adversarial Network (GAN) to replace a medical image such as a CT or MRI image with normal tissue, a different version of the same pathology, or a different pathology, one could utilize several types of GAN architectures, such as the Pix2Pix GAN, CycleGAN, and Conditional GAN (cGAN).

The Pix2Pix GAN, which is based on a conditional GAN framework, is particularly suited for image-to-image translation tasks where there is a paired dataset of images before and after the desired transformation. This model consists of a generator that tries to produce the desired output image from the input image and a discriminator that attempts to distinguish between the real and generated images. The generator is typically a U-Net architecture, which is effective for medical image tasks due to its skip connections that preserve spatial information, while the discriminator is a PatchGAN, which focuses on local image patches for better texture and detail generation.

CycleGAN is another powerful architecture that does not require paired datasets. It works well for tasks where obtaining paired images is difficult. CycleGAN consists of two sets of generators and discriminators. The first generator translates images from domain A (e.g., normal tissue) to domain B (e.g., pathology), and the second generator translates them back from domain B to domain A. Cycle consistency loss ensures that an image translated to another domain and then back to the original domain remains unchanged, enforcing the generators to learn meaningful transformations.

To train these GANs, the following steps can be followed: First, gather a large dataset of medical images, ensuring diversity in normal tissues and various pathologies. Preprocess these images to a consistent size and format and normalize the pixel values to a suitable range (e.g., [-1, 1]). Define the generator and discriminator architectures. For Pix2Pix, the generator can be a U-Net, and the discriminator can be a PatchGAN. For CycleGAN, define two generator-discriminator pairs with ResNet or U-Net generators and PatchGAN discriminators. Implement the adversarial loss, which drives the generator to produce realistic images and the discriminator to distinguish between real and fake images. Additionally, for Pix2Pix, use L1 loss to encourage the generated images to be close to the ground truth. For CycleGAN, implement cycle consistency loss to ensure meaningful mappings between domains.

Train the GANs using the collected dataset. Alternate between training the generator and the discriminator. For each iteration, update the generator to minimize the adversarial loss (and L1 loss for Pix2Pix) and update the discriminator to maximize the adversarial loss. Use techniques such as learning rate scheduling, batch normalization, and instance normalization to stabilize training. Evaluate the trained model using a separate validation set to ensure the generated images are of high quality and medically relevant. Fine-tune the hyperparameters and network architecture as necessary. By using these GAN architectures and following the described training process, one can effectively generate modified medical images that replace original images with normal tissue, different versions of the same pathology, or entirely different pathologies.

In another example, the method further comprises providing a user interface for receiving the selection of the region of interest. The user interface preferably receives the selection of the region of interest using any one of the following: a circular input tool, an ellipsoidal input tool, a rectangular input tool, a lasso tool, a three-dimensional selection tool configured for selecting a volume, and an auto-segmentation tool configured to use an initial selected seed point. All of these tools may facilitate the entry of the region of interest.

In another example, the medical image is any one of the following: a magnetic resonance image, a computed tomography image, a cone beam computed tomography image, a positron emission tomography image, a single-photon emission tomography image, an X-ray image, a digital fluoroscope fluoroscopy image, and a diagnostic ultrasound image. All of these various imaging modalities show the internal anatomical structure of a subject as well as may be used for identifying abnormal anatomical structures.

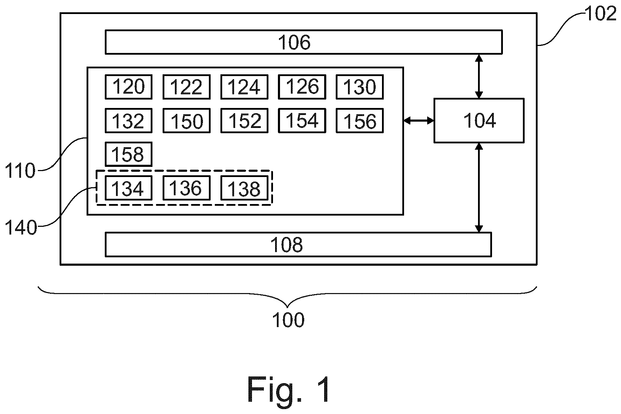

Fig. 1 illustrates an example of a medical system 100. The medical system 100 is formed from a computer 102 that has a computational system 104. The computer 102 may for example have its functionality distributed in one or more computational or computing systems. Likewise, the computational system 104 may represent one or more CPUs or processors located at one or more locations. The computational system 104 is shown as being connected to an optional hardware interface 106 and an optional user interface 108. The hardware interface 106 may for example be used to control other components of the medical system 100 if they are present. For example, the hardware interface 106 could be used to control a magnetic resonance imaging system or a computed tomography system. The user interface 108 may for example be used for controlling the medical system 100 as well as providing renderings of various medical images.

The computational system 104 is further shown as being connected to a memory 110. The memory 110 is intended to represent various types of memory being volatile or non-volatile which may be accessible to the computational system 104. The memory 110 is shown as containing machine-executable instructions 120. The machine-executable instructions 120 enable the computational system 104 to perform various data processing and image processing tasks. The memory 110 is further shown as containing three generative inpainting models 122, 124, and 126. The generative inpainting model 122 could for example be used to replace a region of interest with a normal anatomy for a subject. The generative inpainting model 124 may for example be used for generating abnormal anatomy of the same type. For example, if a tumor is identified in a region of interest it may replace it with a tumor of the same type. The memory 110 is further shown as containing a generative inpainting model 126 that may for example be used to replace the abnormal anatomy in a region of interest with an abnormal anatomy of a different type.

The memory 110 is further shown as containing an acquired medical image 130 that is descriptive of an internal anatomy of a subject. For example, it may be a tomographic medical imaging system which is able to image the internal anatomy of the subject. The memory 110 is further shown as containing a region of interest 132 which identifies a region of the acquired medical system 130 that contains an abnormal anatomical structure. Using generative inpainting or inpainting techniques the generative inpainting models 122, 124, and 126 may be used to generate a variety of generated medical images 134, 136, 138. In this particular example the generated medical image 134 may be an image with the abnormal anatomy removed using the generative inpainting model 122. In this example, the generated medical image 136 may be a generated medical image that shows the same type of abnormal anatomy as is present in the acquired medical image 130. This may for example be accomplished using generative inpainting model 124.

The generated medical image 138 may, in this example, show a different type of abnormal anatomy replaced in the region of interest 132 using the generative inpainting model 126. Together these generated medical images 134, 136, 138, form an ensemble of generated medical images.

In this particular example, three different types of generative inpainting models 122, 124, 126 were used to generate three different images 134, 136, and 138. However, in some examples a single model may be used to generate multiple medical images. For example, the generative inpainting model 122 may be used to make multiple generated medical images 134 with the abnormal anatomy removed. This may for example be averaged to produce an average image which is used as or presented as the generated medical image 134.

These images, 134, 136, 138, may be used in a variety of ways. For example, they may be displayed with the acquired medical image 130 to aid a technician or physician in recognizing or identifying the abnormal anatomy. In other cases this may be used for generating a segmentation. For example, a difference image 150 could be calculated by subtracting the generated medical image 134 and the acquired medical image 130 from each other. As the images would be very close except for the abnormal anatomy, a simple thresholding operation or other technique may be used for generating the segmentation 152. This segmentation 152 could for example be displayed or superimposed on the acquired medical image 130 or it could for example be used to generate therapy system control commands. The very precise segmentation 152 may for example enable better radiotherapy treatment of the abnormal anatomy. The therapy system control commands 154 may for example be used for controlling a therapy system such as a robotic therapy system, an ablation system, or even a radiotherapy system.

In other examples there may also be such things as a tumor progression model 156 that receives the acquired medical image as input and then outputs an image of how the abnormal anatomy changes over a predetermined amount of time. For example, it may represent typical tumor growth. This would then output an optional predicted image 158 which could be used in place of the acquired medical image 130 when determining the segmentation 152. This may for example be very useful in generating the therapy system control commands 154 because it may enable the removal of tissue which is highly likely to become cancerous or have a tumor in the future.

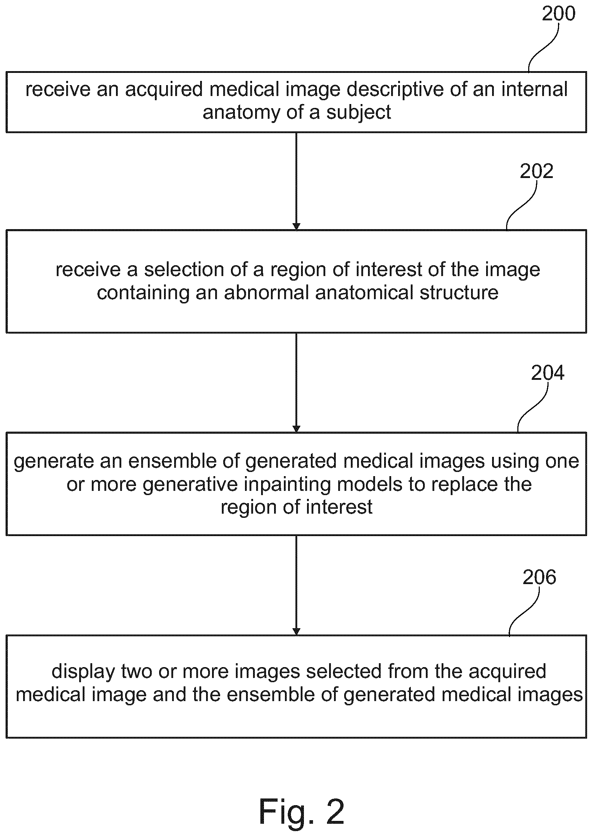

Fig. 2 shows a flowchart which illustrates a method of operating the medical system 100 of Fig. 1. In step 200, the acquired medical image 130 is received. The acquired medical image 130 is descriptive of an internal anatomy of a subject. In step 202 the selection of a region of interest 132 is received. The region of interest 132 contains an abnormal anatomical structure and is located within the acquired medical image 130. In step 204, an ensemble of generated medical images 140 is generated using one or more generative inpainting models 122, 124, 126, to replace the region of interest 132. In step 206, two or more images selected from the acquired medical image 130 and the ensemble of generated medical images 140 are displayed.

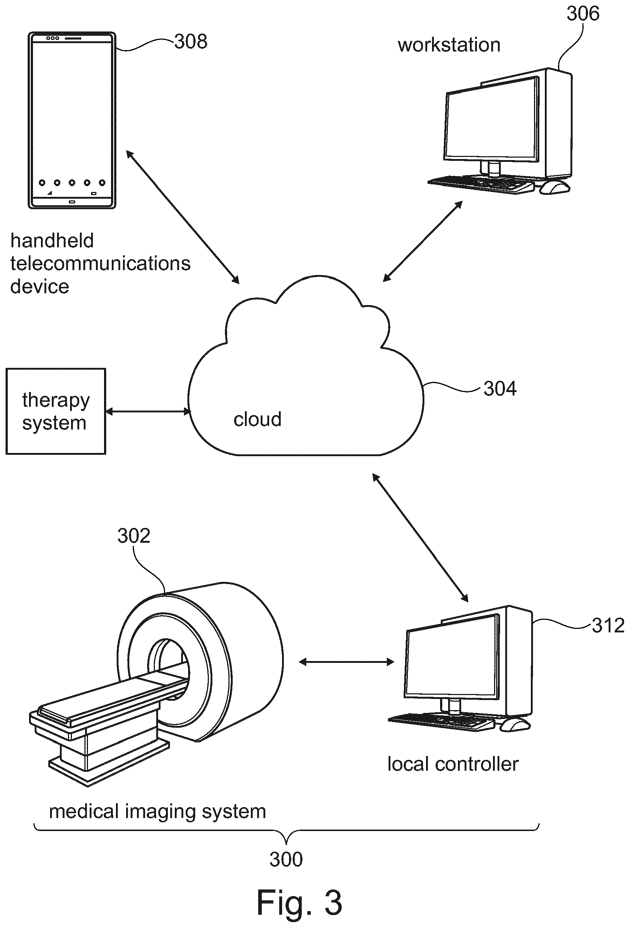

Fig. 3 illustrates a further example of a medical system 300. The medical system 300 comprises a medical imaging system 302, a cloud-based computing system 304, a radiology workstation 306, a handheld telecommunications device 308, a therapy device 310, and a local controller 310. The functionality of the computer 102 depicted in Fig. 1 may be distributed amongst the various devices 302, 304, 306, 308, 310, and 312. The cloud-based computing system 304 could be made up of networked servers and/or virtual machines available over the internet or other network.

The therapy system control commands 154 could be sent to control the therapy system 310.

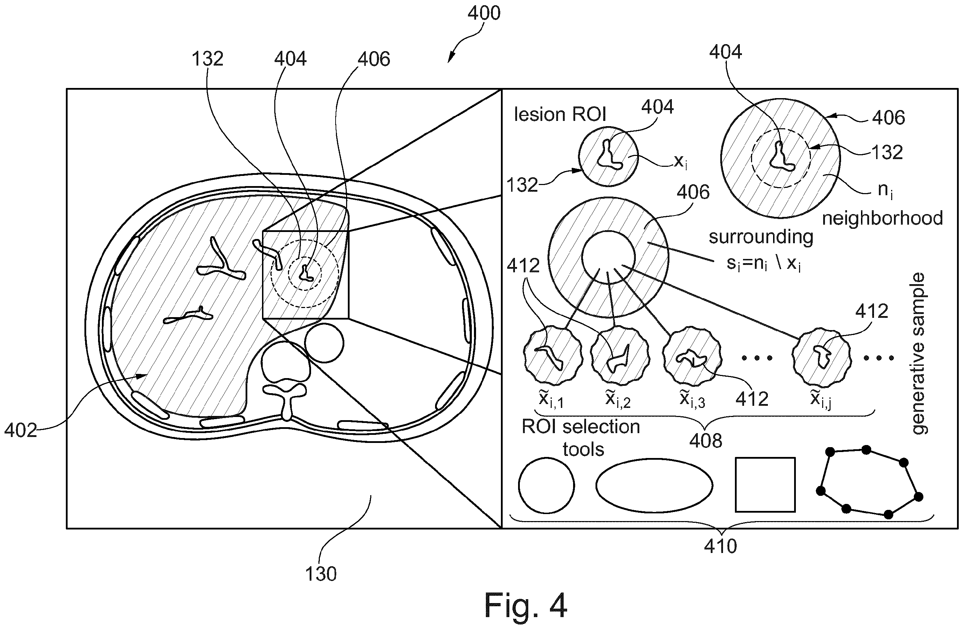

Fig. 4 shows an example of a user interface 400, which is used for selecting the region of interest 132. The user interface 400 displays the acquired medical image 130, which shows the internal anatomy of the subject 402. Within the image there is an abnormal anatomy 404, which has been indicated by the region of interest 132. There are a number of region of interest selection tools for 410, which can be used for selecting the region of interest 132. In this particular example, once the region of interest 132 is defined, there is a neighborhood 406 surrounding the region of interest 132. In, for example, generative inpainting model 124, a number of iterative samples 408 can be used to replace the region of interest 132. In this case, the generative samples 408 have synthetic normal anatomies 412 inpainted into them.

In Fig. 4, given initial ROI(s) *xᵢ* 132, where the index i can run over multiple lesions/ROI(s), one may computes a neighboring image region *nᵢ* 406, which encloses the lesion ROI *xᵢ,* such that *sᵢ* =*nᵢ \ xᵢ* in set notation. I.e., *sᵢ* denotes the surrounding region without the lesion voxels, whereas *nᵢ* includes the lesions voxels x;. The neighboring region can simply be selected by simple region growing algorithms, such that they safely contain the initially selected ROI itself and grow up to a certain heuristic size providing for a certain tolerance margin. Such a size heuristic can for example be a threefold maximal diameter of the initial lesion *xᵢ*. Further any such generated regions, can for example also be constrained by an additional organ segmentation, such that for example not only the lesion but also its neighboring region is fully part of one organ such as the liver.

And any ROI can be in 2D, 3D or 4D (if temporal data exists).

Based on the lesions *xᵢ* and their surroundings *sᵢ*, one uses a generative inpainting model to predict several samples for the original lesion ROI *xᵢ* by simply masking it out. This allows to generate a batch of examples for this region which are artificially synthesized. We denote these newly generated examples as *x̃_{i,j}*, sampled from the conditional distribution *p*(*x*|*sᵢ*). Here, i runs over the individual lesions ROI(s), and j runs over the newly generated lesions image patches based on the generative inpainting solution. Such generative models have been recently established in the literature using latent diffusion models.

The output from this inpainting module *x̃_{i,j}* 408 can be viewed in a separate viewing element using a dedicated browsing mode or can be used to generate an aggregated average view by marginalizing over all created realizations. The number realizations that are created can initially set to a reasonable default value of N = 10, but also be changed by the user of the viewing application.

Further the learned inpainting model can exist in various flavors, either being pre-trained exclusively on healthy tissues, giving predictions of how the lesions ROI would look without pathology, or being exclusively trained on diseased data, to show the reader other examples of similar lesions in that given anatomical / protocol context.

Last, using the conditional distribution p based on the healthy pre-training, one can use the created ensemble {*x̃*_{*i*,*j*} } to generate an average realization and subtract this from the initially masked ROI region *xᵢ* to get an enhanced lesion visualization / representation. The viewing UI can create a dedicated / highlighted view for this task. One may refer to this as a generative background subtraction (GBS).

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,

Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: medical system
- 102: computer
- 104: computational system
- 106: hardware interface
- 108: user interface
- 110: memory
- 120: machine executable instructions
- 122: generative inpainting model (normal anatomy)
- 124: generative inpainting model (alternative abnormal anatomy of same type)
- 126: generative inpainting model (replace abnormal anatomy with different type)
- 130: acquired medical image
- 132: region of interest
- 134: generated medical image (abnormal anatomy removed)
- 136: generated medical image (shows same type of abnormal anatomy)
- 138: generated medical image (shows different type of abnormal anatomy)
- 140: ensemble of generated medical images
- 150: difference image
- 152: image segmentation
- 154: therapy system control commands
- 156: tumor progression model
- 158: predicted image
- 200: receiving an acquired medical image descriptive of an internal anatomy of a subject
- 202: receiving a selection of a region of interest of the acquired medical image containing an abnormal anatomical structure
- 204: generating an ensemble of generated medical images using one or more generative inpainting models to replace the region of interest
- 206: displaying two or more images selected from the acquired medical image and the ensemble of generated medical images
- 300: medical system
- 302: medical imaging system
- 304: cloud computing system
- 306: radiology workstation
- 308: handheld telecommunication device
- 310: therapy system
- 312: local controller
- 400: user interface
- 402: internal anatomy of subject
- 404: abnormal anatomy
- 406: neighborhood surrounding region of interest
- 408: generative sample
- 410: ROI selection tools

## Claims

1. A computer implemented method of medical imaging, the method comprising:
- receiving (200) an acquired medical image (130) descriptive of an internal anatomy of a subject (402);
- receiving (202) a selection of a region of interest (132) of the acquired medical image containing an abnormal anatomical structure (404);
- generating (204) an ensemble (140) of generated medical images (134, 136, 138) using one or more generative inpainting models (122, 124, 126) to replace the region of interest; and
- displaying (206) two or more images selected from the acquired medical image and the ensemble of generated medical images.

2. The computer implemented method of claim 1, wherein the one or more generative inpainting models comprises a normal anatomy generative model configured for generating normal anatomical structures in the region of interest, wherein the ensemble of generative medical images comprises an artificial abnormal structure free image generated using the normal anatomy generative model, and wherein the artificial abnormal structure free image is preferably generated using a combination of multiple images output by the normal anatomy generative model.

3. The computer implemented method of claim 2, wherein the method further comprises:
- generating a difference image (150) by subtracting the acquired medical image and the artificial abnormal structure free image from each other; and
- generate an image segmentation (152) of the abnormal anatomical structure using the difference image.

4. The computer implemented method of claim 3, wherein the method further comprises displaying a superposition of the image segmentation on the acquired medical image.

5. The computer implemented method of claim 4, wherein the abnormal anatomical structure is one or more tumors.

6. The computer implemented method of claim 5, wherein the method further comprises generating a predicted image (158) in response to inputting the acquired medical image into a tumor progression model (156) configured for predicting tumor growth after a predetermined time period, wherein the predicted image is used for determining the image segmentation instead of the acquired medical image.

7. The computer implemented method of any one of claims 2 through 6, wherein the method further comprises generating therapy system control commands (154) using the acquired medical image and the image segmentation, wherein the therapy control commands are configured for controlling a therapy system (310) to modify the abnormal anatomical structure.

8. The computer implemented method of claim 7, wherein the method comprises controlling the therapy system with the therapy control commands.

9. The computer implemented method of any one of the preceding claims, wherein the one or more generative inpainting models further comprises an alternative inpainting model configured for replacing the region of interest with a same type of abnormal anatomical structure.

10. The computer implemented method of any one of the preceding claims, wherein the one or more generative inpainting models further comprises an alternative inpainting model configured for replacing the region of interest with at least one different type of abnormal anatomical structure.

11. The computer implemented method of any one of the preceding claims, wherein the one or more generative inpainting models comprises any one of the following: diffusion inpainting models, GAN inpainting models, and combinations thereof.

12. The computer implemented method of any one of the preceding claims, wherein method comprises providing a user interface for receiving the selection of the region of interest, wherein the user interface preferably receives the selection of the region of interest using any one of the following: a circular input tool, an ellipsoidal input tool, a rectangular input tool, a lasso tool, a three-dimensional selection tool configured for selecting a volume, and an auto segmentation tool configured to use an initial selected seed point.

13. The computer implemented method of any one of the preceding claims, wherein the medical image is any one of the following: a magnetic resonance image, a computed tomography image, a cone beam computed tomography image, a positron emission tomography image, a single photon emission tomography image, an X-ray image, a digital fluoroscopy image, and a diagnostic ultrasound image.

14. A computer program product comprising a computer-readable storage medium having computer-readable program code embodied therewith, said computer-readable program code being configured to implement the method of any one of claims 1 through 13.

15. A medical system (100, 300) comprising:
- a memory (110) storing machine executable instructions and one or more generative inpainting models (!22, 124, 126);
- a computational system (104), wherein execution of the machine executable instructions causes the computational system to:
- receive (200) an acquired medical image (130) descriptive of an internal anatomy of a subject (402);
- receive (202) a selection of a region of interest (132) of the acquired image containing an abnormal anatomical structure (404);
- generate (204) an ensemble (140) of generated medical images (134, 136, 138) using one or more generative inpainting models (122, 124, 126) to replace the region of interest; and
- display (206) two or more images selected from the acquired medical image and the ensemble of generated medical images.
